(19) Europäisches Patentamt
European Patent Office
Office européen des brevets

(11) **EP 4 390 787 A1**

(12) **EUROPÄISCHE PATENTANMELDUNG**

(43) Veröffentlichungstag:
**26.06.2024   Patentblatt 2024/26**

(21) Anmeldenummer: **22216441.0**

(22) Anmeldetag: **23.12.2022**

(51) Internationale Patentklassifikation (IPC):
**G06Q 10/04** *(2023.01)*      **G06Q 50/22** *(2024.01)*
**G16H 40/40** *(2018.01)*

(52) Gemeinsame Patentklassifikation (CPC):
**G06Q 10/04; G06Q 50/22; G16H 40/63**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Paul Hartmann AG**
**89522 Heidenheim (DE)**

(72) Erfinder:
• **JÄSCHKE, Natalie**
**89522 Heidenheim (DE)**
• **LIEBER, Diana**
**89522 Heidenheim (DE)**

(74) Vertreter: **Paul Hartmann AG**
**Patents & Licensing**
**Paul-Hartmann-Straße 12**
**89522 Heidenheim (DE)**

(54) **VERFAHREN ZUR BESTIMMUNG MINDESTENS EINES WECHSELZEITPUNKTS EINES ABSORBIERENDEN ARTIKELS**

(57) Die vorliegende Erfindung betrifft ein Verfahren zur Bestimmung mindestens eines Wechselzeitpunkts eines absorbierenden Artikels, wobei ein erster Abschnitt des Verfahrens unter Verwendung einer Empfängereinheit sowie einer Vielzahl absorbierender Artikel, in denen je ein Feuchtigkeitssensor, je eine Zeitmessvorrichtung und je eine Transmittereinheit vorhanden ist, durchgeführt wird und folgende Schritte umfasst: a) wiederholtes Erfassen von Datenpunkten jeder der von einem Nutzer oder Nutzerkollektiv getragenen absorbierenden Artikel mittels des in dem Artikel vorhandenen Feuchtigkeitssensors und der in dem Artikel vorhandenen Zeitmessvorrichtung, wobei das Erfassen der Datenpunkte während eines Erfassungszeitraums erfolgt, und wobei der Erfassungszeitraum mindestens einen, bevorzugt mehrere Messzeiträume umfasst, b) Übertragen der Datenpunkte mittels der jeweiligen Transmittereinheit an die Empfängereinheit, und wobei ein zweiter Abschnitt des Verfahrens unter Verwendung einer Verarbeitungseinheit, welche mit der Empfängereinheit verbunden ist, durchgeführt wird und folgende Schritte umfasst: a) Bilden mehrerer Datenreihen, welche die in jeweils einem einzigen Messzeitraum empfangenen Datenpunkte enthalten, und b) Bestimmung des Wechselzeitpunkts für die Nutzer oder das Nutzerkollektiv, von denen in Schritt a) Datenpunkte erfasst wurden, wobei die Bestimmung auf einer statistischen Auswertung der Datenreihen beruht und/oder eine statistische Auswertung der Datenreihen umfasst.

Abbildung 4

EP 4 390 787 A1

**Beschreibung**

Die vorliegende Erfindung betrifft ein computerimplementiertes Verfahren zur Bestimmung mindestens eines Wechselzeitpunkts eines absorbierenden Artikels.

EP3747415A1 oder US2011263952A1 offenbaren Inkontinenzprodukte, bei denen Sensordaten, die im Zusammenhang mit einem Miktionsereignis aufgenommen werden, an einen Empfänger übermittelt werden, um einen Wechsel des Produktes vor einem wahrscheinlichen Auslaufen zu initiieren.

In WO13095231 A1 wird eine Methode zur Vorhersage von Miktionsereignissen verwendet, die darauf beruht, dass das Trinkverhalten der Nutzer beobachtet wird. Aufgrund der aufgenommenen Flüssigkeitsmenge wird das Miktionsverhalten vorhergesagt. Dies hat den Nachteil, dass weitere Parameter, teilweise aufwendig, beobachtet werden müssen, um eine Vorhersage machen zu können.

Grundsätzlich würde es den Arbeitsablauf in Pflegestationen erleichtern, wenn dem Personal jederzeit eine Empfehlung eines geeigneten Zeitpunktes zum Wechseln absorbierender Artikel bei den Nutzern zur Verfügung stehen würde.

Die der Erfindung zugrunde liegende Aufgabe besteht darin, die Erfassung des Miktionsverhaltens von Nutzern zu verbessern und somit die Planbarkeit des Personal- und Materialeinsatzes zu erleichtern.

Die zugrundeliegende technische Aufgabe wird gelöst durch ein computerimplementiertes Verfahren zur Bestimmung mindestens eines Wechselzeitpunkts eines absorbierenden Artikels,
wobei ein erster Abschnitt des Verfahrens unter Verwendung einer Empfängereinheit sowie einer Vielzahl absorbierender Artikel, in denen je ein Feuchtigkeitssensor, je eine Zeitmessvorrichtung und je eine Transmittereinheit vorhanden ist, durchgeführt wird und folgende Schritte umfasst:

a) wiederholtes Erfassen von Datenpunkten jeder der von einem Nutzer oder Nutzerkollektiv getragenen absorbierenden Artikel mittels des in dem Artikel vorhandenen Feuchtigkeitssensors und der in dem Artikel vorhandenen Zeitmessvorrichtung, wobei das Erfassen der Datenpunkte während eines Erfassungszeitraums erfolgt, und wobei der Erfassungszeitraum mindestens einen, bevorzugt mehrere Messzeiträume umfasst,
b) Übertragen der Datenpunkte mittels der jeweiligen Transmittereinheit an die Empfängereinheit,

und wobei ein zweiter Abschnitt des Verfahrens unter Verwendung einer Verarbeitungseinheit, welche mit der Empfängereinheit verbunden ist, durchgeführt wird und folgende Schritte umfasst:

c) Bilden mehrerer Datenreihen, welche die in jeweils einem einzigen Messzeitraum empfangenen Datenpunkte enthalten,
d) Bestimmung des Wechselzeitpunkts für die Nutzer oder das Nutzerkollektiv, von denen in Schritt a) Datenpunkte erfasst wurden, wobei die Bestimmung auf einer statistischen Auswertung der Datenreihen beruht und/oder eine statistische Auswertung der Datenreihen umfasst.

Der Zeitpunkt, für den ein Wechseln des jeweiligen absorbierenden Artikels empfohlen wird, wird als Wechselzeitpunkt (W) bezeichnet. Durch die Vorhersage des Wechselzeitpunkts können die Planbarkeit des Personal- und Materialeinsatzes erleichtert werden.

Der Wechselzeitpunkt wird wahlweise für einen einzelnen Nutzer oder ein Kollektiv von Nutzern, beispielsweise alle Nutzer in einer Pflegeeinrichtung oder einer Station, bestimmt. Die Empfehlung des Wechselzeitpunkts basiert auf einer statistischen Auswertung von Datenpunkten, die bei den einzelnen Nutzern oder Nutzerkollektiven erfasst wurden.

Bei dem erfindungsgemäßen Verfahren wird der Wechselzeitpunkt für solche Nutzer oder Nutzerkollektive bestimmt, bei denen vorher Datenpunkte erfasst wurden.

Wurden somit die Datenpunkte nur bei einem einzigen Nutzer erfasst, so kann der Wechselzeitpunkt nurfür diesen Nutzer bestimmt werden. Wurden die Datenpunkte dagegen bei einem Nutzerkollektiv erfasst, so kann der Wechselzeitpunkt sowohl für einen Nutzer, der dem Kollektiv angehört, als auch für das gesamte Kollektiv bestimmt werden.

Allerdings wäre es im Zusammenhang mit einer Weiterentwicklung der Erfindung (nicht beansprucht) grundsätzlich denkbar, die für ein Nutzerkollektiv (bei dem Datenpunkte erfasst wurden) erfolgte Bestimmung des Wechselzeitpunktes auch auf ein anderes Nutzerkollektiv (bei dem keine Datenpunkte erfasst wurden) anzuwenden, sofern dieses andere Nutzerkollektiv eine hinsichtlich der im Zusammenhang mit dem Miktionsverhalten relevanten Einflussgrößen wie zum Beispiel Alter, Geschlecht, Gesundheitszustand oder Tagesablauf ähnliche Zusammensetzung aufweist. Bei einer solchen Übertragung von einem Nutzerkollektiv auf ein anderes wird jedoch die Genauigkeit der Bestimmung des Wechselzeitpunktes vermindert sein.

Jeder Datenpunkt enthält einen Messwert, der mit einem Sättigungsgrad des absorbierenden Artikels korrespondiert, und einem Messzeitpunkt (S), zu dem der Messwert erfasst wurde. Der Sättigungsgrad ist ein Maß dafür, zu welchem Anteil der maximalen Aufnahmekapazität des absorbierenden Artikels zu einem Messzeitpunkt gefüllt ist bzw. welches absolute Flüssigkeitsvolumen der absorbierende Artikel zum Messzeitpunkt fasst. Der Sättigungsgrad kann in einer geeigneten physikalischen Größe oder Maßeinheit erfasst und/oder angegeben werden, beispielsweise in Prozent (relative Sättigung bezogen auf die maximale Aufnahmekapazität des absorbierenden Artikels) oder in ml (absolutes Volumen der in dem absorbierenden Artikel zum Messzeitpunkt vorhandenen Körperflüssigkeit).

Die Messwerte werden mittels des Feuchtigkeitssensors erfasst. Vorzugsweise ist der Feuchtigkeitssensor ein kapazitiver Feuchtigkeitssensor oder ein resistiver Feuchtigkeitssensor. Die Messzeitpunkte werden mittels der Zeitmessvorrichtung erfasst.

Eine Datenreihe wird aus Datenpunkten gebildet. Eine Datenreihe wird dabei jeweils für einen einzigen Nutzer in einem Messzeitraum (m) gleicher oder unterschiedlicher Länge aufgenommen. Der Begriff "Messzeitraum" ist im Rahmen dieser Erfindung zu verstehen als ein Zeitraum einer bestimmten Länge, welcher bei $\tau_m$ beginnt und bei $\tau_{m+1}$ endet. Bevorzugt beträgt die Länge des Messzeitraums einer Datenreihe 24 Stunden. Alternativ kann die Länge des Messzeitraums einer Datenreihe auch einen anderen dem Fachmann als geeignet erscheinenden Zeitraum betragen, beispielsweise 2 Tage, 5 Tage oder 7 Tage. Bevorzugt ist der jeweilige Messzeitraum jeder der Datenreihen gleich lang.

Ein Tragezeitraum (*a*) bezeichnet die Zeit des Tragens eines bestimmten absorbierenden Artikels, vom Anlegen des absorbierenden Artikels an einen Nutzer bis zum Abnehmen dieses absorbierenden Artikels. Eine Tragedatenreihe wird aus den während eines jeweiligen Tragezeitraums aufgenommenen Datenpunkten des jeweiligen Nutzers gebildet. Eine Datenreihe umfasst bevorzugt eine Vielzahl von Tragedatenreihen eines einzelnen Nutzers.

Der Erfassungszeitraum ist der gesamte Zeitraum, in dem Datenpunkte aufgenommen werden. Bevorzugt wird daher der Erfassungszeitraum ein ganzzahliges Vielfaches länger sein als der Messzeitraum. Der Erfassungszeitraum kann mindestens die Länge eines Messzeitraums haben. Ebenfalls kann der Erfassungszeitraum mindestens die Länge von 7 Messzeiträumen haben. Alternativ kann der Erfassungszeitraum mindestens die Länge von 30 Messzeiträumen haben. In einer Ausführungsform kann die Länge des Erfassungszeitraums durch das Hinzufügen weiterer Messzeiträume erweitert werden. In einer anderen Ausführungsform kann der Erfassungszeitraum auf eine bestimmte Anzahl an Messzeiträumen begrenzt sein. Bevorzugt umfasst der Erfassungszeitraum dann die jüngsten Messzeiträume.

Die mindestens jeweils eine Tragedatenreihe umfassenden Datenreihen werden bevorzugt mittels der Verarbeitungseinheit gebildet. Die einzelnen Tragedatenreihen können vollständig oder teilweise in der Datenreihe enthalten sein. Vorzugsweise sind die Datenpunkte der Datenreihen zeitlich aufsteigend oder absteigend geordnet. Weiter bevorzugt werden Tragedatenreihen derart aneinandergefügt und unterteilt, dass die gebildeten Datenreihen sich jeweils über einen Messzeitraum gleicher Länge erstrecken. Insbesondere werden Tragedatenreihen so aneinandergefügt, dass die gebildeten Datenreihen sich jeweils über einen Messzeitraum von je einem Tag erstrecken und weiter insbesondere der früheste Messzeitpunkt jeder Datenreihe für jeden Tag gleich ist. Alternativ können Tragedatenreihen so aneinandergefügt werden, dass die gebildeten Datenreihen sich über zwei oder mehr Tage erstrecken. Dabei ist der früheste Messzeitpunkt jeder Datenreihe bevorzugt zur gleichen Uhrzeit, insbesondere zur gleichen Uhrzeit am gleichen Wochentag.

Die Datenpunkte werden vorzugsweise ununterbrochen während des gesamten jeweiligen Tragezeitraums aufgenommen. Weiter bevorzugt werden die Datenpunkte während des gesamten jeweiligen Tragezeitraums in periodischen Abständen aufgenommen. Bevorzugt sind die periodischen Abstände nicht größer als 30 min, weiter bevorzugt nicht größer als 10 min, weiter bevorzugt sind Abstände nicht größer als 1 min, weiter bevorzugt nicht größer als 1 s.

Bevorzugt beinhalten alle Datenreihen die gleiche Anzahl an Datenpunkten. In einer anderen bevorzugten Ausführungsform kann die Anzahl der Datenpunkte in einer Datenreihe variieren. Weitere Punkte zwischen zwei Datenpunkten können mittels Interpolation ermittelt werden. Interpolation kann beispielsweise verwendet werden, um verschiedene Datenreihen miteinander zu vergleichen bzw. um über diese wie nachfolgend näher beschrieben zu mitteln, falls der zeitliche Abstand der Datenpunkte zwischen den Datenreihen variiert und/oder die Datenpunkte zu anderen Zeitpunkten aufgenommen werden.

Bevorzugt beruht die statistische Auswertung auf den in mehreren Messzeiträumen erfassten Daten. Solchenfalls sind die mehreren Messzeiträume bevorzugt einander zeitlich nacheinander angeordnet. Alternativ kann die statistische Auswertung auf mehreren Datenreihen verschiedener Nutzer während des gleichen Messzeitraums basieren. Als weitere Alternative kann die statistische Auswertung auf mehreren Datenreihen verschiedener Nutzer über verschiedenen Messzeiträumen beruhen.

Bevorzugt umfasst das computerimplementierte Verfahren eine Mittelwertbildung. Die Mittelwertbildung kann für eine Teilmenge der Datenreihen erfolgen. Weiter bevorzugt umfasst die statistische Auswertung eine Mittelwertbildung zur Bildung einer Durchschnittsdatenreihe. In einer bevorzugten Ausführungsform umfasst die Bildung der Durchschnittsdatenreihe die Mittelwertbildung über die Datenreihen mehrerer Messzeiträume. Weiter bevorzugt wird über die Datenreihen verschiedener Messzeiträume und/oder verschiedener Nutzer gemittelt. Hierzu werden die Datenreihen $f_{k,p}(t)$ eines Nutzers p in einem Messzeitraum kaufsummiert und mittels der Gesamtzahl P der Nutzer und der Gesamtzahl N der Messzeiträume wie folgt normiert um die Durchschnittsdatenreihe $F_{av}(t)$ zu erhalten.:

$$F_{av}(t) = 1/(N \cdot P) \sum_{k=1}^{N} \sum_{p=1}^{P} f_{k,p}(t)$$

N und P sind hierbei positive ganze Zahlen. Über N kann der Erfassungszeitraum festgelegt werden. Für N = 1 und P > 1 wird die Durchschnittsdatenreihe mehrerer Nutzer in einem einzigen Messzeitraum berechnet. Für N > 1 und P = 1 wird die Durchschnittsdatenreihe eines einzigen Nutzers über mehrere Messzeiträume berechnet.

In einer Ausführungsform umfasst die statistische Auswertung eine gewichtete Mittelwertbildung, vorzugsweise

$$F_{av}(t) = \frac{1}{P \sum_{k=1}^{N} \exp(-bk)} \sum_{p=1}^{P} \sum_{k=1}^{N} f_{k,p}(t) \exp(-bk)$$

Die Zerfallskonstante b, bei der es sich um eine positive reelle Zahl handelt, bestimmt, wie stark die Datenreihen von weiter zurückliegenden Messzeiträumen die Durchschnittsdatenreihe $F_{av}(t)$ beeinflussen. Je älter die Datenreihe ist, desto größer ist der Index k. Höhere Werte von b führen zu einer geringeren Wichtung vergangener Datenreihen.

Geeignete Werte für b können beispielsweise bei 0.1 bis 5.0 liegen. Falls die Zerfallskonstante b einen Wert von Null aufweist, werden alle Datenreihen bei der Berechnung der Durchschnittsdatenreihe gleichgewichtet und die Ausführungsformen der Mittelwertbildung stimmen überein. Die Definition der anderen Variablen stimmt mit der Definition der vorigen Ausführungsform der Mittelwertbildung überein. In einer anderen Ausführungsform wird nur über Datenreihen gemittelt, welche nach einem Mittelungskriterium ausgewählt werden. Das Mittelungskriterium kann ein bestimmter Wochentag oder ein anderes wiederkehrendes Ereignis sein. Beispielsweise können die Datenreihen aller Montage gemittelt werden. Diese Ausführungsform hat den Vorteil, dass einem strukturierten Tagesablauf eines Nutzers bzw. periodisch wiederauftretenden Ereignissen in einer Pflegeeinrichtung Rechnung getragen werden kann.

In einer Ausführungsform kann der Erfassungszeitraum konstant sein und damit die Gesamtzahl N der Messzeiträume ebenfalls ein konstanter Wert sein. N könnte beispielsweise mindestens die Länge von 7 Messzeiträumen haben. Alternativ kann der Erfassungszeitraum mindestens die Länge von 30 Messzeiträumen haben. In einer anderen Ausführungsform kann die Länge des Erfassungszeitraums durch das Hinzufügen weiterer Messzeiträume erweitert werden. Werden Datenpunkte in weiteren Messzeiträumen aufgenommen, so können sie zur Mittelwertbildung hinzugezogen werden. Der Endwert N wird somit um die Anzahl der hinzugefügten Datenreihen erhöht.

In einer bevorzugten Ausführungsform wird die Durchschnittsdatenreihe $F_{av}(t)$ geglättet. Zur Glättung der Durchschnittsdatenreihe $F_{av}(t)$ wird bevorzugt eine lineare Transformation auf die Durchschnittsdatenreihe $F_{av}(t)$ angewandt. In einer weiter bevorzugten Ausführungsform umfasst die Glättung der Durchschnittsdatenreihe $F_{av}(t)$ einen gleitenden Mittelwert oder einen Savitzky-Golay Filter. In einer anderen bevorzugten Ausführungsform umfasst die Glättung der Durchschnittsdatenreihe $F_{av}(t)$ einen Modified Sinc Kernel, einen Gaußschen Kernel oder Whittaker-Hendersonsmoothing.

In einer alternativen Ausführungsform kann auch die Datenreihe oder die Datenreihen geglättet werden. Die Datenreihe oder die Datenreihen kann nach einer der mit Bezug zur Glättung der Durchschnittsdatenreihe genannten Methoden geglättet werden.

In einer bevorzugten Ausführungsform umfasst das computerimplementierte Verfahren eine Unterteilung der Durchschnittsdatenreihe $F_{av}(t)$ in Wechselzeiträume. Jeder der Wechselzeiträume entspricht einem Abschnitt der Durchschnittsdatenreihe, bzw. der geglätteten Durchschnittsdatenreihe. Innerhalb eines Wechselzeitraums wird ein Wechselzeitpunkt bestimmt. Die Wechselzeiträume überschneiden sich nicht. Die Wechselzeiträume können auf verschiedene, im Folgenden beschrieben, Arten bestimmt werden.

In einer bevorzugten Ausführungsform umfasst das computerimplementierte Verfahren zur Vorhersage zur Bestimmung mindestens eines Wechselzeitpunkts mindestens eine Bestimmung eines lokalen Extremums der Durchschnittsdatenreihe, bzw. der geglätteten Durchschnittsdatenreihe. Bevorzugt werden lokale Minima und/oder lokale Maxima als lokale Extrema bestimmt. Die lokalen Maxima geben die wahrscheinlichste Zeit für das Erreichen der Kapazitätsgrenze der absorbierenden Artikel in dem zwischen zwei lokalen Minima definierten Zeitraum an. Mittels der lokalen Minima können daher die Wechselzeiträume, bevorzugt als Zeitraum zwischen zwei aufeinanderfolgenden lokalen Minima, festgelegt werden. Solchenfalls kann hierbei die Datenreihe oder die Durchschnittsdatenreihe in Wechselzeiträume unterteilt werden, welche durch jeweils zwei Extrema begrenzt sind, wobei die Extrema lokale Minima sind.

Lokale Extrema können beispielsweise mittels der ersten rechts- und linksseitigen Ableitung an der Stelle $t_i$ bestimmt werden:

$$\text{Maximum: } \big(g(t_{i+1}) - g(t_i)\big)/(t_{i+1} - t_i) < 0 \; \wedge \; \big(g(t_i) - g(t_{i-1})\big)/(t_i - t_{i-1}) > 0$$

$$\text{Minimum: } \big(g(t_{i+1}) - g(t_i)\big)/(t_{i+1} - t_i) > 0 \; \wedge \; \big(g(t_i) - g(t_{i-1})\big)/(t_i - t_{i-1}) < 0$$

Hierbei kann es sich bei g um eine Datenreihe oder um die Durchschnittsdatenreihe handeln. Bevorzugt handelt es sich bei g um die geglättete Durchschnittsdatenreihe.

In einer anderen Ausführungsform kann die Unterteilung der Durchschnittsdatenreihe $F_{av}(t)$ in Wechselzeiträume mittels Ausgleichsrechnung erfolgen. Diese Unterteilung kann folgende Schritte umfassen: Eine erste geeignete Wahrscheinlichkeitsverteilung kann ausgewählt werden und an zumindest einen ersten Teilbereich der Durchschnittsdatenreihe angepasst werden. Eine zweite geeignete Wahrscheinlichkeitsverteilung kann ausgewählt werden und an zumindest einen zweiten, weiteren Teilbereich der Durchschnittsdatenreihe angepasst werden. Die Wahrscheinlichkeitsverteilungen können normiert oder nicht normiert sein. Der Beginn eines Wechselzeitraums ist dann durch den ersten Punkt der Durchschnittsdatenreihe oder durch den Schnittpunkt der ersten und zweiten Wahrscheinlichkeitsverteilung gegeben. Das Ende eines Wechselzeitraums ist dann durch den Endpunkt der Durchschnittsdatenreihe oder durch den Schnittpunkt der ersten und zweiten Wahrscheinlichkeitsverteilung gegeben.

Die Wahrscheinlichkeitsverteilung kann beispielsweise eine Gaußfunktion sein. Hier liegt das Maximum der angepassten Wahrscheinlichkeitsverteilung auf dem einem Mittelwert $\mu$.

Ebenso kann eine Verteilung mit einer Schiefe ungleich Null verwendet werden, insbesondere einer Schiefe kleiner Null (linksschiefe Verteilung).

In einer ersten Ausführungsform zur Bestimmung des Wechselzeitpunkts wird ein Zeitpunkt innerhalb eines Wechselzeitraumes mittels eines Faktors $\alpha_n$ gewählt, wobei der Wechselzeitraum mittels Extremum-Bestimmung oder mittels Ausgleichsrechnung bestimmt wurde. $\alpha_n$ ist ein Faktor zwischen 0 und 1. Der Zeitpunkt kann bei $T_n + \alpha_n \Delta T_n$ liegen, wobei $T_n$ der Zeitpunkt des Beginns des $n$-ten Wechselzeitraums ist und $\Delta T_n$ die Zeitdifferenz zwischen dem Beginn dieses Wechselzeitraums und dem Ende desselben Wechselzeitraums. Der festgelegte Zeitpunkt kann bei $T_n + 0.5\Delta T_n$ bis $T_n + \Delta T_n$ liegen. Bevorzugt liegt der festgelegte Zeitpunkt bei $T_n + 0.6\Delta T_n$ bis $T_n + 0.9\Delta T_n$. Besonders bevorzugt ist der festgelegte Zeitpunkt bei $T_n + 0.7\Delta T_n$ bis $T_n + 0.8\Delta T_n$.

Der festgelegte Zeitpunkt kann für jeden Wechselzeitraums auf gleiche Weise nach $T_n + \alpha_n \cdot \Delta T_n$ festgelegt werden mit dem gleichen Faktor $\alpha_n = \alpha \, \forall_n$. Alternativ kann $\alpha_n$ für jeden Wechselzeitraum eingestellt werden. Die Festlegung des $\alpha_n$ für einzelne Wechselzeiträume ist vor allem dann sinnvoll, wenn die einzelnen Wechselzeiträume oder die Umstände während diesen Wechselzeiträumen sich stark unterscheiden. $\alpha_n$ kann beispielsweise von der Verfügbarkeit des Pflegepersonals abhängen. Ebenfalls kann die Streuung der Maxima der einzelnen Datenreihen innerhalb des Wechselzeitraums die Festlegung des $\alpha_n$ beeinflussen.

In einer zweiten Ausführungsform wird der Wechselzeitpunkt, der zwischen dem Beginn des Wechselzeitraums und dem Ende desselben Wechselzeitraums liegt, mittels eines Histogramms bestimmt.

In einer Ausführungsform werden Datenpunkte einer Datenreihe in einem zuvor festgelegten Wertebereich und in einem ausgewählten Zeitbereich als Histogramm aufgetragen. Beispielsweise werden Datenpunkte, deren Messwerte in einem ausgewählten Bereich von Sättigungsgraden in einem Wechselzeitraum liegen, als Histogramm aufgetragen. Bevorzugt soll hier ein Bereich gewählt werden, der hohe Sättigungsgrade umfasst, die allerdings noch nicht einen vollständig gesättigten absorbierenden Artikel repräsentieren, beispielsweise 80 % relative Sättigung bezogen auf die maximale Aufnahmekapazität des absorbierenden Artikels. So verbleibt dem Anwender, beispielsweise einer Pflegekraft, Zeit zu handeln, bevor die maximale Aufnahmekapazität des absorbierenden Artikels überschritten ist und der absorbierende Artikel ausläuft. Wird der n-te Wechselzeitraum, beginnend mit dem Zeitpunkt $T_n$ betrachtet, so gibt das Quantil der Verteilung $P((T_n, t_W]) \geq p$ an, zu welchem Zeitpunkt $t_W$, der in dem $n$-ten Wechselzeitraum liegt, ein Anteil p der Ereignisse in dem festgelegten Wertebereich eingetreten ist.

Beispielsweise kann die untere Grenze des ausgewählten Bereichs von Sättigungsgraden so gewählt werden, dass sie einem Sättigungsgrad von über 40 % relative Sättigung bezogen auf die maximale Aufnahmekapazität des absorbierenden Artikels entspricht. Bevorzugt entspricht die untere Grenze des ausgewählten Bereichs von Sättigungsgraden einem Sättigungsgrad über 50 %, weiter bevorzugt über 55 %, weiter bevorzugt über 60 % relative Sättigung bezogen auf die maximale Aufnahmekapazität des absorbierenden Artikels. Die obere Grenze des ausgewählten Bereichs von Sättigungsgraden wird bevorzugt so gewählt, dass sie einem Sättigungsgrad unter 95 %, weiter bevorzugt unter 90 % relative Sättigung bezogen auf die maximale Aufnahmekapazität des absorbierenden Artikels entspricht. Weiter bevorzugt entspricht die obere Grenze des ausgewählten Bereichs von Sättigungsgraden einem Sättigungsgrad unter 85 %, weiter bevorzugt von höchstens 80 % relative Sättigung bezogen auf die maximale Aufnahmekapazität des absorbierenden Artikels.

Die Bestimmung des Wechselzeitpunkts kann eine Kombination der Methoden der ersten Ausführungsform mittels des Faktors $\alpha_n$ zur Festlegung des Wechselzeitpunkts und der zweiten Ausführungsform mittels Histogramm zur Festlegung des Wechselzeitpunkts umfassen. Bevorzugt werden beide Methoden zur Festlegung des Wechselzeitpunkts herangezogen. Mittels des Vergleichs der beiden Ergebnisse kann eine Qualität der Bestimmung des Wechselzeitpunkts ermittelt werden. Je weniger die beiden Ergebnisse voneinander abweichen, desto besser ist die Bestimmung des Wechselzeitpunkts.

In einer Ausführungsform kann das computerimplementierte Verfahren zur Bestimmung mindestens eines Wechselzeitpunkts eines absorbierenden Artikels mehrere Wechselzeitpunkte zeitlich ordnen. Bei den mehreren Wechselzeitpunkten kann es sich um Vorhersagen für einen Nutzer oder mehrere Nutzer handeln. Mittels der zeitlich geordneten mehreren bestimmten Wechselzeitpunkte kann eine Pflegereihenfolge für Pflegekräfte festgelegt werden. Die Pflegereihenfolge bestimmt in welcher Reihenfolge die Nutzer betreut werden. In einer bevorzugten Ausführungsform wird der Aufenthaltsort des jeweiligen Nutzers verwendet, um eine Pflegereihenfolge festzulegen. Der Aufenthaltsort eines Nutzers kann mittels eines Ortssensors, wie GPS, bestimmt werden. Beispielsweise erfolgt der Wechselvorgang der absorbierenden Artikel eines ersten Nutzers und eines zweiten Nutzers, deren jeweilige Wechselzeitpunkte nur eine kurze Zeitspanne auseinanderliegen, direkt nacheinander, wenn die räumliche Distanz zwischen dem ersten Nutzer und dem zweiten Nutzer kleiner als eine vorbestimmte Grenzdistanz ist. Ein dritter Nutzer, dessen Wechselzeitpunkt zwischen den Wechselzeitpunkten des ersten Nutzers und des zweiten Nutzers liegt, wird direkt davor oder danach betreut, wenn die räumliche Distanz zwischen dem dritten Nutzer und mindestens einem der ersten oder zweiten Nutzer größer als die vorbestimmte Grenzdistanz ist.

Wird das computerimplementierte Verfahren zur Bestimmung mindestens eines Wechselzeitpunkts eines absorbierenden Artikels während mehrerer Messzeiträume durchgeführt, so werden bevorzugt die Ergebnisse der Datenreihen der verschiedenen Messzeiträume verglichen. Es kann das Miktionsverhalten von Nutzern bezüglich bestimmter Parameter, wie beispielsweise Miktionsvolumen oder Anzahl der Miktionen, verfolgt werden. Dadurch können Änderungen des Miktionsverhaltens und Ausreißer ermittelt werden. Ausreißer sind einzelne Messwerte, die aus der Reihe fallen, die also außerhalb eines vorbestimmten tolerierten Wertebereiches der jeweiligen Messung liegen. Ausreißer spielen in dem bevorzugten Verfahren mit Mittelwertbildung und anschließendem Glätten eine nur geringe Rolle für die Bestimmung des Wechselzeitpunkts. Allerdings können Ausreißer ein Anzeichen für ein außergewöhnliches Ereignis sein. Ein solches außergewöhnliches Ereignis kann beispielsweise auf einen fehlerhaften Sensor, einen Benutzerfehler (z.B. Sensor wird in Flüssigkeit getaucht) oder auch auf eine Änderung des Gesundheitszustandes des Nutzers zurückzuführen sein. In einer bevorzugten Ausführungsform wird im Falle eines Ausreißers ein Warnsignal ausgegeben.

Eine Änderung des Miktionsverhaltens des Nutzers oder einer Gruppe von Nutzern kann mittels einer beobachteten Änderung der Kurvenform der Datenreihe oder der Durchschnittsdatenreihe ermittelt werden. Die Datenreihe oder die Durchschnittsdatenreihe ausgewählter Messzeiträume wird dabei verglichen mit einer Durchschnittsdatenreihe, welche durch Mittelwertbildung über eine Vielzahl von Messzeiträumen bestimmt wird. Die Änderung der Kurvenform kann beispielsweise mittels einer Differenz zwischen einer Tageskurve eines Nutzers und einer zeitgemittelten Kurve dieses Nutzers bestimmt werden. Die Änderung des Miktionsverhaltens kann beispielsweise anhand einer Änderung der Anzahl der lokalen Maxima und/oder lokalen Minima in verschiedenen Messzeiträumen erkannt werden.

Die Erfindung betrifft weiterhin ein System zur Bestimmung mindestens eines Wechselzeitpunkts eines absorbierenden Artikels. Das System umfasst eine Empfängereinheit und eine Verarbeitungseinheit, welche mit der Empfängereinheit verbunden ist, sowie eine Vielzahl absorbierender Artikel, in denen je ein Feuchtigkeitssensor, je eine Zeitmessvorrichtung und je eine Transmittereinheit vorhanden ist. Wobei mittels des Systems ein Verfahren ausgeführt wird, dessen erster folgende Schritte umfasst:

a) wiederholtes Erfassen von Datenpunkten jeder der von einem Nutzer oder Nutzerkollektiv getragenen absorbierenden Artikel mittels des in dem Artikel vorhandenen Feuchtigkeitssensors und der in dem Artikel vorhandenen Zeitmessvorrichtung, wobei das Erfassen der Datenpunkte während eines Erfassungszeitraums erfolgt, und wobei der Erfassungszeitraum mindestens einen, bevorzugt mehrere Messzeiträume umfasst,
b) Übertragen der Datenpunkte mittels der jeweiligen Transmittereinheit an die Empfängereinheit,

- und wobei ein zweiter Abschnitt des Verfahrens unter Verwendung der Verarbeitungseinheit, durchgeführt wird und folgende Schritte umfasst:

c) Bilden mehrerer Datenreihen, welche die in jeweils einem einzigen Messzeitraum empfangenen Datenpunkte enthalten,
d) Bestimmung des Wechselzeitpunkts für die Nutzer oder das Nutzerkollektiv, von denen in Schritt a) Datenpunkte erfasst wurden, wobei die Bestimmung auf einer statistischen Auswertung der Datenreihen beruht und/oder eine statistische Auswertung der Datenreihen umfasst.

In einer bevorzugten Ausführungsform umfasst das System eine Ausgabeeinheit. Die Datenreihen bzw. die Durchschnittsdatenreihen sowie die bestimmten Wechselzeitpunkte können mittels der Ausgabeeinheit dargestellt werden. Bevorzugt handelt es sich bei der Ausgabeeinheit um einen Bildschirm einer externen Benutzereinheit. Bevorzugt ist die externe Benutzereinheit eine mobile Einheit, wie beispielsweise ein Smartphone. Alternativ kann es sich bei der Ausgabeeinheit um einen Bildschirm, welcher an eine stationäre Recheneinheit angeschlossen ist, handeln.

Weiter betrifft die Erfindung ein Computerprogramm zur Bestimmung mindestens eines Wechselzeitpunkts eines absorbierenden Artikels, umfassend Befehle, die bei der Ausführung des Programms durch einen Computer diesen veranlassen, das Verfahren zur Bestimmung mindestens eines Wechselzeitpunkts eines absorbierenden Artikels auszuführen.

Weiter betrifft die Erfindung ein computerlesbares Speichermedium, umfassend Befehle, die bei der Ausführung durch einen Computer diesen veranlassen, das Verfahren zur Bestimmung mindestens eines Wechselzeitpunkts eines absorbierenden Artikels auszuführen.

Beispiel

Zum besseren Verständnis der vorliegenden Erfindung und ihrer Vorteile wird nun auf die folgende beispielhafte Ausführungsform in Verbindung mit den zugehörigen Abbildungen 1 bis 4 verwiesen.

Die Abbildungen zeigen:

Abbildung 1: Schematische und nicht maßstäbliche Darstellung eines Messzeitraums
Abbildung 2: Mehrere beispielhafte Datenreihen
Abbildung 3: Eine Durchschnittsdatenreihe
Abbildung 4: Eine geglättete Durchschnittsdatenreihe mit lokalen Maxima und lokalen Minima

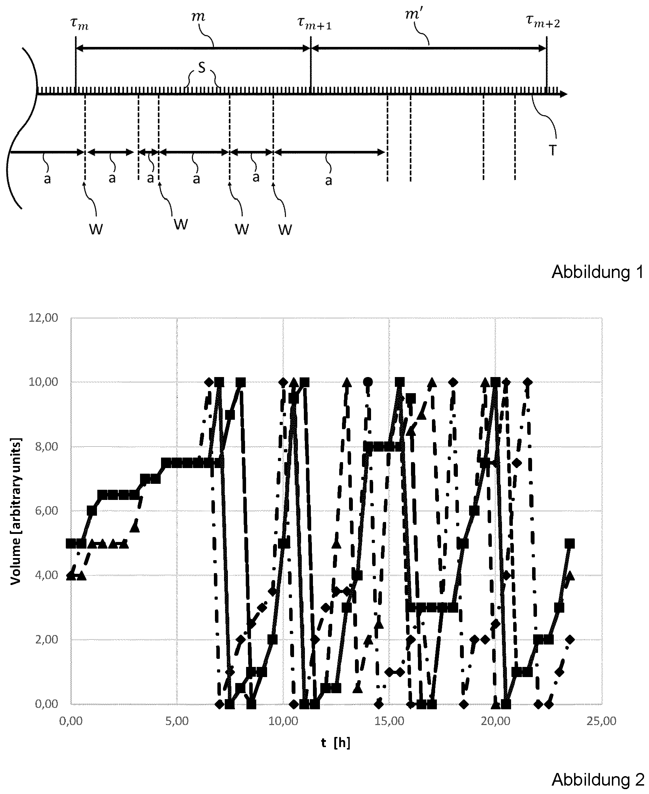

In Abbildung 1 ist schematisch, nicht maßstäblich, ein Messzeitraum $m$ dargestellt. Gestrichelte vertikale Linien markieren eine beispielhafte Anzahl von mehreren Wechselzeitpunkten W von absorbierenden Artikeln eines einzigen Nutzers entlang einer horizontalen aufgetragenen Zeitachse T. Zu einem Messzeitpunkt S wird ein Messwert erfasst. Ein Datenpunkt enthält den Messwert und den Messzeitpunkt S. Der Zeitraum zwischen zwei Wechselzeitpunkten W ist jeweils ein Tragezeitraum $a$. Während des Tragezeitraums $a$ wird jeweils eine hier nicht dargestellte Tragedatenreihe aufgenommen, die aus den in dem jeweiligen Tragezeitraum $a$ aufgenommenen Datenpunkten gebildet wird. Die Datenpunkte von mehreren aufeinanderfolgenden Tragedatenreihen zu einen Messzeitraum $m$, beginnend mit $\tau_m$ und endend mit $\tau_{m+1}$, bilden eine in Abbildung 1 nicht dargestellte und in Abbildung 2 näher beschriebene Datenreihe. In Abbildung 1 schließt sich an den Messzeitraum $m$ ein beispielhafter weiterer Messzeitraum $m$ beginnend mit $\tau_{m+1}$ und endend mit $\tau_{m+2}$ desselben Nutzers an. In einem Messzeitraum $m$ kann auch eine unvollständiger Tragezeitraum $a$ enthalten sein.

Abbildung 2 zeigt mehrere Datenreihen, die anhand verschiedener Linienarten voneinander unterscheidbar dargestellt sind.

Es werden beispielhafte Datenpunkte verwendet. Diese beispielhaften Datenpunkte können Messdaten eines Feuchtigkeitssensors eines absorbierenden Artikels oder bearbeitete Messdaten sein, bei denen beispielsweise auf übliche Weise Rauschen herausgerechnet wurde oder bei denen die Messkurven verschiedener absorbierender Artikel normiert wurden. Die Normierung kann erfolgen, wenn beispielsweise Artikel, welche unterschiedliche Absorptionskapazitäten, Größen oder Auslaufverhalten aufweisen, verwendet werden. Eine Normierung oder relative Angabe des Sättigungsgrads erleichtert es, Messkurven derart verschiedener Artikel miteinander zu vergleichen. Es wird der Wert 10 gewählt, um anzuzeigen, dass eine maximale Aufnahmekapazität des Artikels erreicht wurde und ein Wechsel notwendig ist. Ein Wert von 0 bedeutet, dass der Artikel trocken ist.

Ein negativer Sprung, also ein abrupter Abfall der Kurvenform einer Datenreihe, repräsentiert einen Wechsel des gebrauchten absorbierenden Artikels zu einem neuen absorbierenden Artikel bei einem Nutzer.

Die den einzelnen Datenreihen zugrunde liegenden Datenpunkte in Abbildung 2 können während mehreren einander zeitlich nacheinander angeordneten Messzeiträumen von je einem Tag oder während mehreren einander zeitlich nacheinander angeordneten Messzeiträumen von mehreren Nutzern an einem einzigen Tag erfasst werden. Ebenfalls ist eine Kombination von Datenreihen mehrerer Messzeiträumen und Datenreihen mehrerer Nutzer möglich.

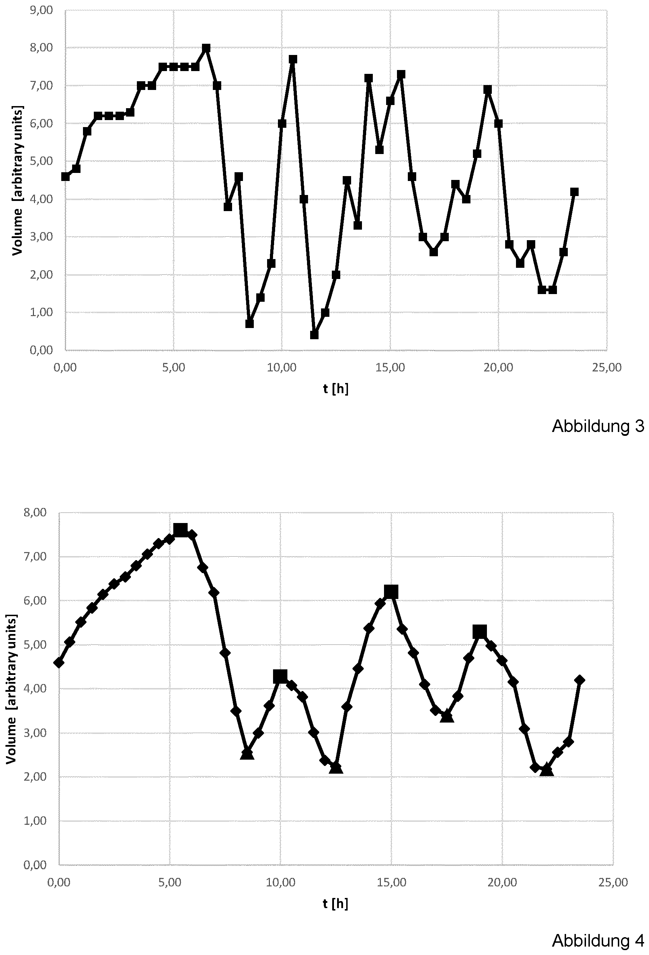

Aus den Datenreihen wird eine in Abbildung 2 nicht dargestellte Durchschnittsdatenreihe berechnet. Jeder Datenpunkt geht mit gleicher Gewichtung in die Berechnung der Durchschnittsdatenreihe ein. Die Durchschnittsdatenreihe dieses Beispiels ist in Abbildung 3 dargestellt.

Die Durchschnittsdatenreihe wurde mittels eines gleitenden Mittelwertes geglättet und anschließend lokale Maxima und lokale Minima bestimmt. Die geglättete Durchschnittsdatenreihe ist in Abbildung 4 gezeigt. Zwei aufeinanderfolgende lokalen Minima (hervorgehoben durch Dreiecke) grenzen einen Wechselzeitraum ein. Ebenfalls sind die lokalen Maxima (hervorgehoben durch Quadrate) in Abbildung 4 dargestellt.

Ausgehend von einem lokalen Minimum $T_n$ wird ein nachfolgender festgelegter Zeitpunkt, der einen empfohlenen Zeitpunkt des Wechselns eines absorbierenden Artikels angibt mittels $T_n + 0.7\Delta T_n$ bestimmt. Der so bestimmte Wechselzeitpunkte wird mittels einer Ausgabeeinheit, beispielsweise ein Bildschirm eines Smartphones, ausgegeben. Die auf diese Weise bestimmten Wechselzeitpunkte können die Planbarkeit des Personal- und Materialeinsatzes erleichtern. So kann ein höherer Personaleinsatz vorgesehen werden, wenn in einem Zeitfenster in der Zukunft viele der auf diese Weise bestimmten Wechselzeitpunkte liegen. Diese Häufung solcher Wechselzeitpunkte kann mittels eines Zusammentreffens der erfindungsgemäß bestimmten Wechselzeitpunkte vieler Nutzer zustande kommen und/oder durch externe Gegebenheiten, wie beispielsweise einen Mahlzeitenplan einer Pflegeeinrichtung, gegeben sein. Gleichzeitig kann in Zeitfenstern, in denen mit einer niedrigen Anzahl von auf die hier offenbare Weise bestimmten Wechselzeitpunkten gerechnet wird, ein geringerer Personaleinsatz vorgesehen werden. Die durch die Vorhersagen erhaltenen Informationen können so zu einem gezielteren Einsatz von Pflegekräften führen, der besser an die Bedürfnisse der Nutzer angepasst ist und beitragen kann, eine Überforderung von Pflegekräften zu verringern.

**Patentansprüche**

1. Computerimplementiertes Verfahren zur Bestimmung mindestens eines Wechselzeitpunkts eines absorbierenden Artikels,
wobei ein erster Abschnitt des Verfahrens unter Verwendung einer Empfängereinheit sowie einer Vielzahl absorbierender Artikel, in denen je ein Feuchtigkeitssensor, je eine Zeitmessvorrichtung und je eine Transmittereinheit vorhanden ist, durchgeführt wird und folgende Schritte umfasst:

a) wiederholtes Erfassen von Datenpunkten jeder der von einem Nutzer oder Nutzerkollektiv getragenen absorbierenden Artikel mittels des in dem Artikel vorhandenen Feuchtigkeitssensors und der in dem Artikel vorhandenen Zeitmessvorrichtung, wobei das Erfassen der Datenpunkte während eines Erfassungszeitraums erfolgt, und wobei der Erfassungszeitraum mindestens einen, bevorzugt mehrere Messzeiträume umfasst,
b) Übertragen der Datenpunkte mittels der jeweiligen Transmittereinheit an die Empfängereinheit,
- und wobei ein zweiter Abschnitt des Verfahrens unter Verwendung einer Verarbeitungseinheit, welche mit der Empfängereinheit verbunden ist, durchgeführt wird und folgende Schritte umfasst:

c) Bilden mehrerer Datenreihen, welche die in jeweils einem einzigen Messzeitraum empfangenen Datenpunkte enthalten,
d) Bestimmung des Wechselzeitpunkts für die Nutzer oder das Nutzerkollektiv, von denen in Schritt a) Datenpunkte erfasst wurden, wobei die Bestimmung auf einer statistischen Auswertung der Datenreihen beruht und/oder eine statistische Auswertung der Datenreihen umfasst.

2. Computerimplementiertes Verfahren zur Bestimmung mindestens eines Wechselzeitpunkts eines absorbierenden Artikels nach Anspruch 1, wobei eine Datenreihe aus Datenpunkten gebildet wird, die für einen einzigen Nutzer aufgenommen wird.

3. Computerimplementiertes Verfahren zur Bestimmung mindestens eines Wechselzeitpunkts eines absorbierenden Artikels nach Anspruch 1 oder 2, wobei die statistische Auswertung eine Mittelwertbildung zur Bildung einer Durchschnittsdatenreihe umfasst.

4. Computerimplementiertes Verfahren zur Bestimmung mindestens eines Wechselzeitpunkts eines absorbierenden Artikels nach Anspruch 3, wobei die Bildung der Durchschnittsdatenreihe die Mittelwertbildung über die Datenreihen mehrerer Messzeiträume umfasst.

5. Computerimplementiertes Verfahren zur Bestimmung mindestens eines Wechselzeitpunkts eines absorbierenden Artikels nach einem der Ansprüche 3 oder 4, wobei die Bildung der Durchschnittsdatenreihe die Mittelwertbildung über die Datenreihen mehrerer Nutzer umfasst.

6. Computerimplementiertes Verfahren zur Bestimmung mindestens eines Wechselzeitpunkts eines absorbierenden Artikels nach mindestens einem der Ansprüche 3 bis 5, wobei die Mittelwertbildung eine gewichtete Mittelwertbildung ist.

7. Computerimplementiertes Verfahren zur Bestimmung mindestens eines Wechselzeitpunkts eines absorbierenden Artikels nach mindestens einem der vorherigen Ansprüche, wobei das Verfahren ein Glätten der Datenreihe oder Datenreihen oder ein Glätten der Durchschnittsdatenreihe umfasst.

8. Computerimplementiertes Verfahren zur Bestimmung mindestens eines Wechselzeitpunkts eines absorbierenden Artikels nach mindestens einem der vorhergehenden Ansprüche, wobei das Verfahren eine Bestimmung mindestens eines Extremums umfasst, wobei das mindestens eine Extremum ein lokales Minimum oder ein lokales Maximum sein kann.

9. Computerimplementiertes Verfahren zur Bestimmung mindestens eines Wechselzeitpunkts eines absorbierenden Artikels nach mindestens einem der Ansprüche 5 bis 8, wobei das Verfahren eine Bestimmung mindestens eines Extremums der Durchschnittsdatenreihe umfasst.

10. Computerimplementiertes Verfahren zur Bestimmung mindestens eines Wechselzeitpunkts eines absorbierenden Artikels nach Anspruch 9, wobei die Durchschnittsdatenreihe in Wechselzeiträume unterteilt wird, welche durch jeweils zwei Extrema begrenzt sind, wobei die Extrema lokale Minima sind.

11. Computerimplementiertes Verfahren zur Bestimmung mindestens eines Wechselzeitpunkts eines absorbierenden Artikels nach Anspruch 10, wobei das Verfahren die Erstellung eines Histogramms umfasst, wobei das Histogramm Datenpunkte in einem ausgewählten Wertebereich und in einem ausgewählten Zeitbereich umfasst.

12. Computerprogramm zur Bestimmung mindestens eines Wechselzeitpunkts eines absorbierenden Artikels, umfassend Befehle, die bei der Ausführung des Programms durch einen Computer diesen veranlassen, das Verfahren nach mindestens einem der Ansprüche 1 bis 11 auszuführen.

13. Computerlesbares Speichermedium, umfassend Befehle, die bei der Ausführung durch einen Computer diesen veranlassen, das Verfahren zur Bestimmung mindestens eines Wechselzeitpunkts eines absorbierenden Artikels nach mindestens einem der Ansprüche 1 bis 11 auszuführen.

14. System zur Bestimmung mindestens eines Wechselzeitpunkts eines absorbierenden Artikels umfassend eine Empfängereinheit und eine Verarbeitungseinheit, welche mit der Empfängereinheit verbunden ist, sowie eine Vielzahl absorbierender Artikel, in denen je ein Feuchtigkeitssensor, je eine Zeitmessvorrichtung und je eine Transmittereinheit vorhanden ist, wobei mittels des Systems ein Verfahren ausgeführt wird, dessen erster Abschnitt folgende Schritte umfasst:

a) wiederholtes Erfassen von Datenpunkten jeder der von einem Nutzer oder Nutzerkollektiv getragenen absorbierenden Artikel mittels des in dem Artikel vorhandenen Feuchtigkeitssensors und der in dem Artikel vorhandenen Zeitmessvorrichtung, wobei das Erfassen der Datenpunkte während eines Erfassungszeitraums erfolgt, und wobei der Erfassungszeitraum mindestens einen, bevorzugt mehrere Messzeiträume umfasst,
b) Übertragen der Datenpunkte mittels der jeweiligen Transmittereinheit an die Empfängereinheit,
- und wobei ein zweiter Abschnitt des Verfahrens unter Verwendung der Verarbeitungseinheit, durchgeführt wird und folgende Schritte umfasst:

c) Bilden mehrerer Datenreihen, welche die in jeweils einem einzigen Messzeitraum empfangenen Datenpunkte enthalten,
d) Bestimmung des Wechselzeitpunkts für die Nutzer oder das Nutzerkollektiv, von denen in Schritt a) Datenpunkte erfasst wurden, wobei die Bestimmung auf einer statistischen Auswertung der Datenreihen beruht und/oder eine statistische Auswertung der Datenreihen umfasst.

Abbildung 1

Abbildung 2

Abbildung 3

Abbildung 4

Europäisches Patentamt
European Patent Office
Office européen des brevets

**EUROPÄISCHER RECHERCHENBERICHT**

Nummer der Anmeldung

EP 22 21 6441

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/352794 A1 (CURRAN PETER [AU] ET AL) 12. November 2020 (2020-11-12) * Absätze [0010], [0011], [0037], [0041], [0042], [0072], [0115] – [0124] * ----- | 1-14 | INV. G06Q10/04 G06Q50/22 G16H40/40 |
| X | US 2016/158071 A1 (BARDA DAVID ALBERT [AU] ET AL) 9. Juni 2016 (2016-06-09) * Absätze [0002], [0013], [0016], [0021], [0074], [0109], [0124], [0129] * * Absätze [0135] – [0144], [0150] – [0153], [0214] * ----- | 1-14 | |
| X | US 2017/000655 A1 (MASHIN-CHI HADI [AU] ET AL) 5. Januar 2017 (2017-01-05) * Absätze [0001], [0009] – [0016], [0035] – [0056], [0066], [0075] – [0090], [0116] * ----- | 1-14 | RECHERCHIERTE SACHGEBIETE (IPC) G06Q G16H |

Der vorliegende Recherchenbericht wurde für alle Patentansprüche erstellt

| Recherchenort | Abschlußdatum der Recherche | Prüfer |
|---|---|---|
| München | 1. Juni 2023 | Breidenich, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 21 6441

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-06-2023

| Im Recherchenbericht angeführtes Patentdokument | Datum der Veröffentlichung | Mitglied(er) der Patentfamilie | | Datum der Veröffentlichung |
|---|---|---|---|---|
| US 2020352794 A1 | 12-11-2020 | AU | 2018363877 A1 | 25-06-2020 |
| | | CA | 3084835 A1 | 16-05-2019 |
| | | EP | 3706693 A1 | 16-09-2020 |
| | | US | 2020352794 A1 | 12-11-2020 |
| | | WO | 2019090387 A1 | 16-05-2019 |
| US 2016158071 A1 | 09-06-2016 | AU | 2011267839 A1 | 10-01-2013 |
| | | CA | 2802503 A1 | 22-12-2011 |
| | | DK | 2582341 T3 | 06-06-2016 |
| | | DK | 3064179 T3 | 20-12-2021 |
| | | EP | 2582341 A1 | 24-04-2013 |
| | | EP | 3064179 A1 | 07-09-2016 |
| | | NZ | 603953 A | 27-03-2015 |
| | | US | 2013254141 A1 | 26-09-2013 |
| | | US | 2016158071 A1 | 09-06-2016 |
| | | WO | 2011156862 A1 | 22-12-2011 |
| US 2017000655 A1 | 05-01-2017 | AU | 2016204461 A1 | 19-01-2017 |
| | | US | 2017000655 A1 | 05-01-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3747415 A1
- US 2011263952 A1
- WO 13095231 A1